# EUROPEAN PATENT SPECIFICATION

(11) **EP 1 818 048 B1**
(45) Date of publication and mention of the grant of the patent: **07.01.2009**
(21) Application number: 06250788.4
(22) Date of filing: 14.02.2006
(51) Int. Cl.: A61K 9/20

(54) **Pharmaceutical formulations of aliphatic amine polymers and methods for their manufacture**
Pharmazeutische Zubereitungsform von aliphatischen amin-Polymeren und Verfahren zu deren Herstellung
Compositions pharmceutiques des polymères aminées aliphatiques et procedé pour les produire

(43) Date of publication of application: 15.08.2007
(73) Proprietor: Teva Pharmaceutical Industries, Ltd., 49131 Petah Tiqva (IL)
(72) Inventor: Hrakovsky, Julia, Rosh Ha-Ayin 48057 (IL); Tenengauzer, Ruth, Hod Hasharon 45322 (IL); Ioffe, Alla, Kfar-Saba (IL); Moin-Kotliar, Eleonora, Herzliya 46504 (IL)
(74) Representative: Nachshen, Neil Jacob

(56) References cited:
- EP-A- 0 997 148
- EP-A- 1 153 940
- US-A1- 2002 035 089

## Description

### FIELD OF THE INVENTION

The present invention relates to pharmaceutical compositions comprising aliphatic amine polymers, such as for example Sevelamer HCl, as the active pharmaceutical ingredient and methods of preparing pharmaceutical compositions thereof, such as for example Sevelamer HCl Film Coated Tablets employing wet granulation.

### BACKGROUND OF THE INVENTION

Aliphatic amine polymers are useful as active pharmaceutical ingredients for use in pharmaceutical compositions. A particularly interesting aliphatic amine polymer is Sevelamer, a polymeric phosphate binder intended for oral administration. Sevelamer hydrochloride is a poly(allylamine hydrochloride) crosslinked with epichlorohydrin in which forty percent of the amines are apparently protonated. It is known chemically as poly(allylamine-co-N,N'-diallyl-1,3-diamino-2-hydroxypropane) hydrochloride. Sevelamer hydrochloride is hydrophilic, but insoluble in water. The reported structure of Sevelamer HCl is represented below:

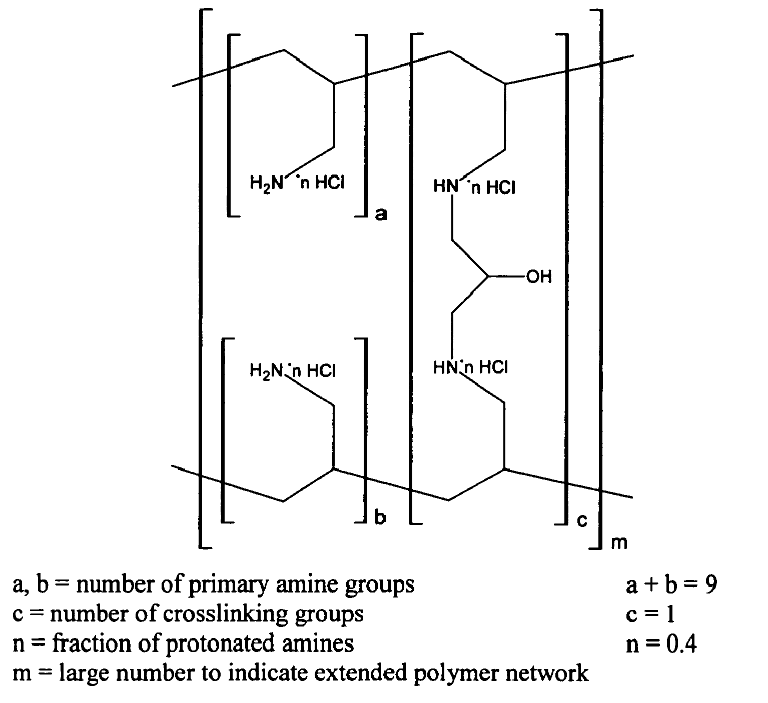

Sevelamer HCl is currently being marketed as RENAGE^{®}, for the treatment of patients with Chronic Kidney Disease (CKD) which are on hemodialysis¹. According to the prescribing information RENAGEL^{®} is indicated for the control of serum phosphorus in such CKD patients. In general, commercially available Sevelamer tablets also contain the following inactive ingredients; hypromellose, diacetylated monoglyceride, colloidal silicon dioxide, and stearic acid.
¹ Renagel^{®} is sold by Genzyme Corporation. The Prescribing Information is available from www.renagel.com/docs/renagel_pi.pdf which describes the 400mg and 800mg tablets as containing the following inactive ingredients: hypromellose, diacetylated monoglyceride, collodial silicon dioxide and stearic acid.

The method of producing a direct compressible composition of a polymer tablet core is described in U.S. Patent No. 6,733,780 B1 and U.S. Patent Application 2005/0260236 A1. These references describe Sevelamer HCl as a product which compressibility is strongly dependent upon the degree of hydration. Apparently, hydrating the polymer to the desired moisture level is considered by those inventors as an essential first step in manufacturing the finished product.

Further, Sevelamer HCl is known to be very hygroscopic and swell upon contact with water. Such swelling of the aliphatic amine polymer Sevelamer complicates formulating the active pharmaceutical ingredient in a pharmaceutical composition. Thus although compressibility is apparently dependant on the degree of hydration, simply adding water to sevelamer results in a swollen material which swollen material is impossible to use to press tablets. The present invention overcomes this problem of swelling of the active pharmaceutical ingredient by providing a more compressible sevelamer formulation within which the water content of sevelamer is not significantly increased compared to the commercially available sevelamer raw material. Even when using a wet granulation method with the present sevelamer formulation, the active pharmaceutical ingredient sevelamer has not swollen significantly. It is noted that commercially available sevelamer raw material is similarly impossible to compress.

### SUMMARY OF THE INVENTION

In one aspect the present invention provides a pharmaceutical composition comprising, a) aliphatic amine polymers, and b) at least one pharmaceutical excipient, wherein the aliphatic amine polymer in the composition comprises wet granulated aliphatic amine polymers, and wherein the aliphatic amine polymer is Sevelamer HCl or Sevelamer Carbonate. The granulation liquid for preparing the wet granulation of aliphatic amine polymers is an ethanol/water solution of about 82% to about 95% ethanol (95%) and about 5% to about 18% water.

Further, there is provided a method of preparing a granular composition comprising wet granulated aliphatic amine polymers comprising the following steps of
a) providing aliphatic amine polymers;
b) providing aa granulating solution , which can optionally contain one or more excipients such as a binder;
c) contacting the granulating solution with the aliphatic amine polymers by preferably spraying it onto the aliphatic amine polymers and one or more excipients and forming a wet granulate, more preferably performed in a fluidized bed drier; and
d) mixing the wet granulate with one or more excipients and forming a final blend, wherein the granulating solution is an ethanol/water solution of about 82% to about 95% ethanol (95%) and about 5% to about 18% water.
Furthermore, the method preferably further comprises the steps of
e) pressing the final blend into tablets.
Alternatively, the method further comprises the step of
e) filling capsules with the final blend.

In another aspect, the present invention provides the above pharmaceutical composition comprising wet granulated aliphatic amine polymers, preferably manufactured by spray granulation techniques, wherein the pharmaceutical composition has a degree of hydration of less than about 7% as measured by Karl Fischer (KF) analysis and is compressible and suitable for tableting, and wherein the aliphatic amine polymer is selected from the group consisting of Sevelamer HCl and Sevelamer carbonate.

In another aspect, the present invention provides the above pharmaceutical compositions for treating a patient suffering from chronic kidney disease comprising administering in combination with hemodialysis a therapeutically effective amount of the Sevelamer HCl.

### DETAILED DESCRIPTION OF THE INVENTION

To achieve an adequate protection against swelling upon contact with water of highly hygroscopic active pharmaceutical ingredients such as aliphatic amine polymers in a pharmaceutical composition is an important aspect in formulating a dosage form of aliphatic amine polymers for use in medical treatments. A preferred aliphatic amine polymer composition for use in the formulations of the present invention is Sevelamer HCl. According to the present invention, formulations of aliphatic amine polymers with a low hydration level and which are highly compressible can be achieved when the aliphatic amine polymers are wet granulated with a granulating solution comprising organic solvents preferably mixed with water optionally containing one or more of a binder. In addition, these formulations of aliphatic amine polymers of the present invention have a lower hydration level compared to that of the Renagel^{®} formulations. Moreover, the aliphatic amine polymers in the pharmaceutical composition of the present invention have a similarly low hydration level when compared to the aliphatic amine polymer raw material prior to its use in such pharmaceutical composition. The wet granulation is preferably achieved by techniques of spray granulation. These pharmaceutical compositions according to the present invention are easily prepared and have good storage properties. The present invention thus provides a pharmaceutical composition comprising aliphatic amine polymers wherein the objective of providing a compressible wet granulated formulation of for example sevelamer with a desired protection against the swelling propensity of the active ingredient in such pharmaceutical compositions. This objective is achieved by using a formulation wherein the aliphatic amine polymers are wet granulated with at least one organic solvent preferably mixed with water, most preferably by spray wet granulation.

In a first approach developing a pharmaceutical composition comprising aliphatic amine polymers, tablets were produced using a conventional direct compression method. However, the material of the aliphatic amine polymer formulation was determined to be not compressible. Secondly, a wet granulation method was attempted using a high shear mixer employing water as the only granulating liquid. Similarly, the method was determined to be not suitable because of swelling of the active material during the granulation process due to the use of water as the only granulating liquid. As a consequence, the material for the aliphatic amine polymer formulation is not compressible. In contrast, it was surprisingly found that using an ethanol/water granulation solution, as for example in a wet granulation method with such ethanol/water solution, is an appropriate method for producing Sevelamer tablets. The spray granulation technique is particularly favored.

One embodiment of the present invention provides a pharmaceutical composition comprising, a) granulated aliphatic amine polymers, and b) at least one pharmaceutical excipient, wherein the aliphatic amine polymer in the composition comprises preferably wet granulated, more preferably spray wet granulated aliphatic amine polymers. Moreover, granulated aliphatic amine polymers are granulated employing a granulating liquid of ethanol/water solution, wherein the granulation liquid comprises about 82% to about 95% ethanol (95%) and about 5% to about 18% water. More preferably, the granulation liquid comprises 95% ethanol and 5% water. Furthermore, the aliphatic amine polymer is Sevelamer HCl or Sevelamer Carbonate, more preferably the aliphatic amine polymer is Sevelamer HCl. Moreover, the pharmaceutical composition comprises preferably from about 60% to about 95% by weight of Sevelamer HCl, more preferably from about 65% to about 85%, most preferably from about 65% to about 80% by weight of Sevelamer HCl. Furthermore, the Sevelamer HCl in the pharmaceutical composition is preferably substantially non-hydrated Sevelamer HCl.

In another embodiment, the present invention provides a pharmaceutical composition comprising spray granulated aliphatic amine polymers, wherein the pharmaceutical composition has a degree of hydration of less than about 7% as measured by Karl Fischer analysis, wherein the aliphatic amine polymer is Sevelamer HCl or Sevelamer Carbonate. More preferably the aliphatic amine polymer used in the process for manufacturing the formulation is a substantially non-hydrated aliphatic amine polymer. Furthermore, in the form of a tablet such pharmaceutical composition preferably has a hardness of at least about 17 Strong Cobb Units (SCU), more preferably from about 17 SCU to about 40 SCU. Further, such tableted pharmaceutical composition of the present invention preferably has a friability of less than about 1%, more preferably less than 0.1 %. Moreover, such tableted pharmaceutical composition of the present invention preferably has a disintegration time in 0.1N HCl of less than about 5 minutes, more preferably from about 2½ to about 3 minutes.

As stated above, granulation solutions/liquids for use in the present invention are ethanol/water solutions wherein the granulation liquid comprises about 82% to about 95% ethanol (95%) and about 5% to about 18% water. More preferably, the granulation liquid comprises 95% ethanol and 5% water.

The pharmaceutical compositions of wet granulated, preferably wet spray granulated, aliphatic amine polymers of the present invention may further contain excipients such as tablet and capsule fillers and diluents (such as microcrystalline cellulose, lactose, starch and tri-basic calcium phosphate), disintegrants (such as starch, croscarmellose sodium, crospovidone and sodium starch glycolate), binders (such as starch, hydroxypropyl methyl cellulose and Povidone), glidant (such as colloidal silicon dioxide), lubricants (such as magnesium stearate, magnesium lauryl sulfate, stearic acid and sodium stearyl fumarate).

More particularly, suitable diluents and fillers for use in the pharmaceutical composition of the present invention include microcrystalline cellulose (e.g. Avicel^{®}), lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, potassium chloride, powdered cellulose, sodium chloride, sorbitol and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydroxyethyl cellulose, hydroxypropyl cellulose (e.g. Klucel^{®}), hydroxypropyl methyl cellulose (e.g. Methocel^{®}), liquid glucose, maltodextrin, methylcellulose, polymethacrylates, povidone (e.g. Povidone PVP K-30, Kollidon^{®}, Plasdone^{®}), pregelatinized starch, sodium alginate and starch. Furthermore, Povidone has been found to be a particularly useful binder. Moreover, this binder is preferably added as a solution in the granulating liquid.

A compacted solid pharmaceutical composition may also include the addition of a disintegrant to the composition. Disintegrants include croscarmellose sodium (e.g. Ac Di Sol^{®}, Primellose^{®}), crospovidone (e.g. Kollidon^{®}, Polyplasdone^{®}), microcrystalline cellulose, polacrilin potassium, powdered cellulose, pregelatinized starch, sodium starch glycolate (e.g. Explotab^{®}, Primoljel^{®}) and starch.

Glidants can be added to improve the flowability of a solid composition before compaction and to improve the accuracy of dosing especially during compaction and capsule filling. Excipients that may function as glidants include colloidal silicon dioxide, magnesium trisilicate, powdered cellulose, and talc.

A lubricant can be added to the composition to reduce adhesion and/or ease the release of the product from e.g. the dye. Lubricants include magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc and zinc stearate.

Other excipients that may be incorporated into the formulation include preservatives, surfactants, antioxidants, or any other excipient commonly used in the pharmaceutical industry.

The solid compositions of the present invention include powders, granulates, aggregates and compacted compositions. The dosages include dosages suitable for oral, buccal, and rectal administration. Although the most suitable administration in any given case will depend on the nature and severity of the condition being treated, the most preferred route of the present invention is oral. The dosages may be conveniently presented in unit dosage form and prepared by any of the methods well known in the pharmaceutical arts.

The pharmaceutical composition of the present invention may be prepared in any dosage form such as a compressed granulate in the form of a tablet for example. Also, uncompressed granulates and powder mixes that are obtained by the method of the present invention in the precompression steps can be simply provided in dosage form of a capsule or sachet. Therefore, dosage forms of pharmaceutical composition of the present invention include solid dosage forms like tablets, powders, capsules, sachets, troches and losenges. The dosage form of the present invention may also be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

Once an aliphatic amine polymer, preferably Sevelamer HCl, solid composition is prepared in accordance with the present invention, it is preferably formulated into pharmaceutical formulations such as conventional dosage forms, including tablets and capsules. Tablets are preferred dosage forms. In addition, the tablets may be coated with an optional cosmetic tablet coating.

The present invention also provides a method of preparing pharmaceutical compositions comprising aliphatic amine polymers selected from the group consisting of Sevelamer HCl and Sevelamer Carbonate, preferably Sevelamer HCl. Preferably, the method of the present invention produces compressed solid dosage forms. There are three well known processes for manufacturing such dosage forms; (i) direct compression, (ii) dry granulation and (iii) wet granulation. There are two well known processes for wet granulation. A wet granulate can be prepared using a mixer and subsequently the wet granulate is dried in order to obtain a dry homogenous granulate. In another method a wet granulate is prepared by spray granulation. In a fluid-bed, spray granulation process, particles and granulate are built up in a fluid bed by spraying a liquid onto fluidized particles. Thus in such process materials are fluidized in the fluid bed dryer and subsequently a solution is sprayed through a nozzle. The choice of processing approach depends upon the properties of the drug and chosen excipients, for example particle size, blending compatibility, density and flowability. For preparing Sevelamer HCl tablets, granulation is preferred, wherein wet granulation being the most preferred with the technique of spray granulation being the method of choice. In addition, the tablets may also be coated to assure ease of swallowing or to provide an elegant appearance.

In one embodiment, the present invention provides a method of preparing a pharmaceutical composition comprising wet granulated aliphatic amine polymers comprising the steps of
a) providing aliphatic amine polymers;
b) wet granulating the aliphatic amine polymers with one or more excipients and forming a pharmaceutical composition, wherein the wet granulation solutions are ethanol/water solutions, of about 82% to about 95% ethanol (95%) and about 5% to about 18% water. Most preferred is a wet granulation solution of 95% ethanol (95%) and 5% water.

In another embodiment, the present invention provides a method of preparing a granular composition comprising spray granulated aliphatic amine polymers comprising the following steps of
a) providing aliphatic amine polymers;
b) preparing a granulating liquid and optionally a binder preferably in the form of a solution;
c) spraying the granulating liquid onto aliphatic amine polymers and one or more excipients and forming a spray granulate; and
d) mixing the spray granulate with one or more excipients and forming a final blend wherein the granulating liquid is an ethanol/water solution of about 82% to about 95% ethanol (95%) and about 5% to about 18% water. Preferably the aliphatic amine polymer is Sevelamer HCl, more preferably the Sevelamer HCl used in the method is substantially non-hydrated Sevelamer HCl.
Preferably, the method further comprises the steps of
e) pressing the final blend into tablets.
Alternatively, the method further comprises the steps of
e) filling capsules with the final blend.

Specifically, the preferred method for the production of a pharmaceutical composition of wet granulated aliphatic amine polymers involves spraying of a granulating liquid or solution onto aliphatic amine polymers. The spraying process preferably uses Fluidized bed technology equipment, where the particles are suspended in a vertical column with a rising air stream. While the particles are fluidized, the granulating solution is sprayed into the column. This spraying can be carried out by any one of three methods; top spray, bottom spray and a "tangential" or powder spray. Preferably the spraying is carried out by a top spray method.

Spray granulation solutions suitable for use in a spray granulated process need to be selected according to requirements some of which are dependent on the physical properties of the active pharmaceutical ingredient. For example the granulation solutions should not alter the physical characteristics of and/or dissolve the active pharmaceutical ingredient. The spray granulation solutions for use in the method of the present invention is an ethanol/water solution, and most preferably a solution of about 82% to about 95% ethanol (95%) and about 5% to about 18% water. Most preferred is a spray granulation solution of 95% ethanol (95%) and 5% water.

The Sevelamer pharmaceutical compositions in the form of film coated tablets prepared by the described method may contain per tablet various amounts of Sevelamer HCl and preferably contain either about 400 mg or about 800 mg of Sevelamer HCl as calculated on an anhydrous basis.

In another embodiment, the present invention provides the above pharmaceutical compositions for treating a patient suffering from chronic kidney disease comprising administering in combination with hemodialysis a therapeutically effective amount of Sevelamer HCl in the pharmaceutical composition.. Patients suffering from chronic kidney disease CKD are frequently treated with hemodialysis. In such patients, which are treated with hemodialysis, it is important to control the phosphorus content in their serum. Sevelamer HCl binds phosphorus and is therefore used as a phosphorus scavenger in the blood stream of patients to control serum phosphorus content. Thus the present invention provides the above pharmaceutical compositions for treating a patient suffering from chronic kidney disease and undergoing hemodialysis treatment comprising administering a therapeutically effective amount of Sevelamer HCl in the pharmaceutical composition. Preferably, the treatment of a patient undergoing hemodialysis comprises administering a therapeutically effective amount of Sevelamer HCl in the pharmaceutical composition comprising wet granulated Sevelamer HCl controls the serum phosphorus content in such patient.

The scope of the invention should not be limited to the working examples, which are for demonstration purposes. One skilled in the art can practice the invention based on the disclosures in the present patent application.

### EXAMPLES

### Renagel^{®} Tablets and Sevelamer HCl active ingredient

The tablets of the following examples 1 and 2 (tablets A and B respectively) were prepared according to the formulations and methods of the invention. Certain physical properties of these tablets A and B were determined as shown in Table 1 of example 3. Some of these physical properties were also determined for a comparative RENAGEL^{®} tablet as presented in Table 3. In addition to the physical properties of tablets A and B some physical properties of the Sevelamer HCl active ingredient as used in the preparations of Examples 1 and 2 were determined. The active ingredient Sevelamer HCl used in the following examples was manufactured by Shasun Chemicals and Drugs Ltd with the following properties as shown in Table 1.

**Table 1: Properties of Sevelamer raw material.**

| Lot No. | Karl Fischer | Loss on drying (LOD) |
|---|---|---|
| RLB 054M0327 | 4.6 % | 11.4% |

It is understood that, while the LOD result is indicative of all the material that can be evaporated or removed by heat or drying, it is the Karl Fischer analysis that determines the water content or level of hydration of the material tested.

### Example 1: Tablet A of 400 mg spray granulated Sevelamer HCl

| Ingredient | *K-35987^{*}* mg/tablet |
|---|---|
| **Part 1** | |
| Sevelamer HCl | 400.0 |
| Povidone | 28.0 |
| Ethanol/Water granulating liquid | |
| **Part 2** | |
| Low-Substituted Hydroxypropyl Cellulose | 177.0 |
| Hydrogenated Vegetable Oil | 10.0 |
| **Part 3** | |
| ⁺Opadry^{®} | 9.45 |

| | |
|---|---|
| ^{*}Batch number ⁺commercially available mix of polymer, plasticizer and opacifying agent | |

The Sevelamer HCl was spray-granulated using a Povidone solution in mixture of ethanol/ water as a granulating liquid. The granulate was blended with Part II ingredients. The final blend was compressed into tablet core and coated using Part III material. The solvents in the granulating liquid were comprised of either a mixture of 95% Ethanol (95%) and 5% water or a mixture of 82% Ethanol (95%) and 18% water.

### Example 2: Tablet B of 800 mg spray granulated Sevelamer HCl

| Ingredient | *K-36180^{*}* mg/tablet |
|---|---|
| **Part 1** | |
| Sevelamer HCl | 800.0 |
| Povidone | 56.0 |
| Ethanol/Water granulating liquid | |
| **Part 2** | |
| Low-Substituted Hydroxypropyl Cellulose | 299.5 |
| Hydrogenated Vegetable Oil | 16.0 |
| **Part 3** | |
| ⁺Opadry^{®} | 39.0 |

| | |
|---|---|
| ^{*}Batch number ⁺commercially available mix of polymer, plasticizer and opacifying agent | |

The Sevelamer HCl was spray-granulated using a Povidone solution in mixture of ethanol/ water as a granulating liquid. The obtained granulate was blended with Part II ingredients. The final blend was compressed into tablet core and coated using Part III material. The solvents in the granulating liquid were comprised of either a mixture of 95% Ethanol (95%) and 5% water or a mixture of 82% Ethanol (95%) and 18% water.

### Example 3: Properties of Sevelamer HCl preparation of Working Examples

The Sevelamer HCl tablets prepared according to the present invention were subjected to various analyses to determine their physical properties. The following Table 2 shows the results thereof and lists the obtained values for the physical properties of the tablets according to examples 1 and 2.

**Table 2: Physical properties of Sevelamer HCl preparations.**

| **Example (Batch No.)** | **Flowability** | **LOD of the granulate** | **Hardness** | **Friability** | **KF** | **Disintegration time (HCl) (min)** |
|---|---|---|---|---|---|---|
| 1 (K-35987) | Very Good | 10.3 | 17 SCU | 0.1% | 5.38 - cores | 2:34 |
| 2 (K-36180) | Very Good | 12.2 | 40 SCU | 0% | 5.35-cores 6.5 - coated tablets | 2:36 |

The obtained granulates have very good or excellent flowability. Further, no swelling of the active material was observed to have occurred during the granulation process. Moreover, the compressed tablets have acceptable physical characteristics and disintegration times. The results obtained by Karl Fischer analysis indicate that tablets are produced without a significant change in the water content or Hydration level as evidenced by the KF value. The preferred hydration value as measured by Karl Fischer analysis can therefore be set at not more than 7%. In the following Table 3 some of the physical properties for the commercially available Renagel^{®} tablets are shown.

**Table 3: Physical properties of Renagel Tablets.**

| **Lot no.** | **KF** | **LOD** |
|---|---|---|
| #643105 | 10.4% | 9.4% |
| #35276A | 9.0% | 8.5% |

It can be seen that the material that can be removed by evaporation from the Renagel^{®} tablets consists almost entirely of water. Evidence hereof is the close similarity between the results obtained from the LOD and KF analyses. In contrast, it can be observed that in the tablets of the present invention the water content or hydration level of the sevelamer in the preparations, as evidenced by the Karl Fischer analysis, is very similar to the levels found in the raw active material received from the supplier.

## Claims

1. A pharmaceutical composition comprising;
a) wet granulated aliphatic amine polymer selected from the group consisting of Sevelamer HCl and Sevelamer Carbonate obtained by using an ethanol/water solution as the granulation solution; and
b) at least one pharmaceutical excipient;
wherein the ethanol/water solution is a solution of about 82% to about 95% ethanol (95%) and about 5% to about 18% water.

2. The pharmaceutical composition according to claim 1, wherein the ethanol/water solution is a solution of 95% ethanol (95%) and 5% water.

3. The composition according to claim 1, wherein the wet granulated aliphatic amine polymers are spray granulated.

4. The pharmaceutical composition according to claim 3, wherein the aliphatic amine polymer is Sevelamer HCl.

5. The pharmaceutical composition according to claim 3 or 4, wherein Sevelamer HCl is substantially non-hydrated and which hydration level is similar in comparison to the hydration level of raw material Sevelamer HCl.

6. The pharmaceutical composition according to any of claims 3-5, wherein the amount of Sevelamer HCl is from about 60% to about 95% of the weight of the composition.

7. The pharmaceutical composition according to any preceding claim, wherein the composition is in a pharmaceutical dosage form.

8. The pharmaceutical composition according to claim 7, wherein the dosage form is a tablet.

9. The pharmaceutical composition according to claim 8, wherein the tablet has a water content of less than 7% as measured by Karl Fischer analysis.

10. The pharmaceutical composition according to claim 8 or 9, wherein the tablet has a hardness of at least about 17 SCU.

11. The pharmaceutical composition according to claim 8, 9 or 10, wherein the tablet has a friability of less than about 1.0%.

12. The pharmaceutical composition according to any of claims 8-11, wherein the tablet has a disintegration time in 0.1N HCl of less than about 5 minutes.

13. The pharmaceutical composition according to any of claims 8-12, wherein the aliphatic amine polymer is Sevelamer HCl.

14. The pharmaceutical composition according to claim 13, wherein the amount of Sevelamer HCl, calculated as anhydrous Sevelamer HCl, is from about 400 mg to about 800 mg in each tablet.

15. The pharmaceutical composition according to claim 7, wherein the dosage form is a capsule.

16. A method of preparing a granular composition comprising wet granulated aliphatic amine polymers comprising the following steps of
a) providing aliphatic amine polymers;
b) preparing a granulating solution or liquid, wherein the granulating solution is an ethanol/water solution of about 82% to about 95% ethanol (95%) and about 5% to about 18% water;
c) spraying the granulating liquid or solution onto aliphatic amine polymers and forming a spray granulate; and
d) mixing the spray granulate with one or more excipients forming a final blend.

17. The method according to claim 16, wherein the granulating solution is an ethanol/water solution prepared from 95% of Ethanol (95%) and 5% water.

18. The method according to any of claims 16-17, wherein the granulating solution or liquid further comprises a binder material.

19. The method according to claim 18, wherein the binder material is dissolved in the granulating solution or liquid

20. The method according to claim 19, wherein the binder material comprises povidone.

21. The method according to any of claims 16-20, wherein the one or more excipient comprises low-substituted Hydroxypropyl Cellulose, hydrogenated vegetable oil and combinations thereof.

22. The method according to any of claims 16-21, wherein spraying of the granulation liquid is carried out in a fluidized bed equipment.

23. The method according to claim 22, wherein spraying of the granulating liquid is carried out by using a spraying method selected from the group consisting of top spray, bottom spray, and tangential/powder spray.

24. The method according to claim 23, wherein the spraying method is the top spray method.

25. The method according to claim 16, further comprising the steps of
e) pressing the final blend into tablets.

26. The method according to claim 25, further comprising the step of
e) coating the tablets.

27. The method according to claim 16, further comprising the steps of e) filling capsules with the final blend.

28. The method according to any of claims 16-27, wherein the aliphatic amine polymer is selected from the group consisting of Sevelamer HCl and Sevelamer Carbonate.

29. The method according to claim 28, wherein the aliphatic amine polymer is Sevelamer HCl.

30. Use of sevelamer HCl in the manufacture of a pharmaceutical composition defined in any of claims 1-15 for the treatment of chronic kidney disease.

31. Use according to claim 30, wherein the treatment with Sevalamer HCl controls serum phosphate.

32. A pharmaceutical composition according to any of claims 1-15 for use in the treatment of chronic kidney disease.

## Patentansprüche

1. Pharmazeutische Zusammensetzung, welche folgendes umfaßt:
a) feuchtgranuliertes aliphatisches Aminpolymer, ausgewählt aus der Gruppe, bestehend aus Sevelamer-HCl und Sevelamer-Carbonat, erhalten unter Verwendung einer Ethanot/Wasser-Lösung als Granulationslösung, und
b) wenigstens einen pharmazeutischen Hilfsstoff,
wobei die Ethanol/Wasser-Lösung eine Lösung von etwa 82% bis etwa 95% Ethanol (95%) und etwa 5% bis etwa 18% Wasser ist.

2. Pharmazeutische Zusammensetzung nach Anspruch 1, wobei die Ethanol/Wasser-Lösung eine Lösung von 95% Ethanol (95%) und 5% Wasser ist.

3. Zusammensetzung nach Anspruch 1, wobei die feuchtgranulierten aliphatischen Aminpolymere sprühgranuliert sind.

4. Pharmazeutische Zusammensetzung nach Anspruch 3, wobei das aliphatische Aminpolymer Sevelamer-HCl ist.

5. Pharmazeutische Zusammensetzung nach Anspruch 3 oder 4, wobei Sevelamer-HCl im wesentlichen nicht hydratisiert ist und das Hydratisierungsniveau im Vergleich zu dem Hydratisierungsniveau von Sevelamer-HCl-Rohmaberial ähnlich ist.

6. Pharmazeutische Zusammensetzung nach einem der Ansprüche 3 bis 5, wobei die Menge an Sevelamer-HCl von etwa 60% bis etwa 95% des Gewichts der Zusammensetzung ausmacht.

7. Pharmazeutische Zusammensetzung nach einem der vorangegangenen Ansprüche, wobei die Zusammensetzung in Form einer pharmazeutischen Dosierungsform vorliegt.

8. Pharmazeutische Zusammensetzung nach Anspruch 7, wobei die Dosierungsform eine Tablette ist.

9. Pharmazeutische Zusammensetzung nach Anspruch 8, wobei die Tablette einen Wassergehalt von weniger als 7% hat, gemessen mittels Karl-Fischer-Analyse.

10. Pharmazeutische Zusammensetzung nach Anspruch 8 oder 9, wobei die Tablette eine Härte von wenigstens etwa 17 SCU hat.

11. Pharmazeutische Zusammensetzung nach Anspruch 8, 9 oder 10, wobei die Tablette eine Brüchigkeit von wenigstens etwa 1,0% hat.

12. Pharmazeutische Zusammensetzung nach einem der Ansprüche 8 bis 11, wobei die Tablette eine Zerfallzeit in 0,1 N HCl von weniger als etwa 5 Minuten hat.

13. Pharmazeutische Zusammensetzung nach einem der Ansprüche 8 bis 12, wobei das aliphatische Aminpolymer Sevelamer-HCl ist.

14. Pharmazeutische Zusammensetzung nach Anspruch 13, wobei die Menge an SevelamerHCl, berechnet als wasserfreies Sevelamer-HCl, in jeder Tablette von etwa 400 mg bis etwa 800 mg beträgt.

15. Pharmazeutische Zusammensetzung nach Anspruch 7, wobei die Dosierungsform eine Kapsel ist.

16. Verfahren zum Herstellen einer granulären Zusammensetzung, welche feuchtgranulierte aliphatische Aminpolymere umfaßt, wobei das Verfahren die folgenden Stufen umfaßt:
a) Bereitstellen von aliphatischen Aminpolymeren,
b) Herstellen einer Granulationslösung oder -flüssigkeit, wobei die Granulationslösung eine Ethanol/Wasser-Lösung von etwa 82% bis etwa 95% Ethanol (95%) und etwa 5% bis etwa 18% Wasser ist,
c) Sprühen der Granulationsflüssigkeit oder -lösung auf aliphatische Aminpolymere und Bilden eines Sprühgranulats und
d) Mischen des Sprühgranulats mit einem oder mehreren Hilfsstoffen unter Bildung eines Endgemischs.

17. Verfahren nach Anspruch 16, wobei die Granulationslösung eine Ethanol/Wasser-Lösung ist, die aus 95% Ethanol (95%) und 5% Wasser hergestellt ist.

18. Verfahren nach einem der Ansprüche 16 bis 17, wobei die Granulationslösung oder -flüssigkeit weiterhin ein Bindermaterial umfaßt.

19. Verfahren nach Anspruch 18, wobei das Bindermaterial in der Granulationslösung oder -flüssigkeit gelöst ist.

20. Verfahren nach Anspruch 19, wobei das Bindermaterial Povidon umfaßt.

21. Verfahren nach einem der Ansprüche 16 bis 20, wobei der eine oder die mehreren Hilfsstoffe gering substituierte Hydroxypropylzellulose, hydriertes Pflanzenöl und Kombinationen davon umfaßt bzw. umfassen.

22. Verfahren nach einem der Ansprüche 16 bis 21, wobei das Sprühen der Granulationsflüssigkeit in einer Wirbelschichteinrichtung durchgeführt wird.

23. Verfahren nach Anspruch 22, wobei das Sprühen der Granulationsflüssigkeit unter Verwendung eines Sprühverfahrens durchgeführt wird, das aus der Gruppe ausgewählt ist, bestehend aus Sprühen von oben ("top spray"), Sprühen von unten ("bottom spray") und Tangential/Pulver-Spray.

24. Verfahren nach Anspruch 23, wobei das Sprühverfahren das Verfahren mit Sprühen von oben ist.

25. Verfahren nach Anspruch 16, welches weiterhin die Stufe umfaßt, bei der man
e) das Endgemisch zu Tabletten preßt.

26. Verfahren nach Anspruch 25, welches weiterhin die Stufe umfaßt, bei der man
e) die Tabletten beschichtet.

27. Verfahren nach Anspruch 16, welches weiterhin die Stufe umfaßt, bei der man
e) Kapseln mit dem Endgemisch füllt.

28. Verfahren nach einem der Ansprüche 16 bis 27, wobei das aliphatische Aminpolymer aus der Gruppe ausgewählt ist, bestehend aus Sevelamer-HCl und Sevelamer-Carbonat.

29. Verfahren nach Anspruch 28, wobei das aliphatische Aminpolymer Sevelamer-HCl ist.

30. Verwendung von Sevelamer-HCl bei der Herstellung einer pharmazeutischen Zusammensetzung, wie sie in einem der Ansprüche 1 bis 15 definiert ist zur Behandlung von chronischer Nierenerkrankung.

31. Verwendung nach Anspruch 30, wobei die Behandlung mit Sevelamer-HCl das Serumphosphat kontrolliert.

32. Pharmazeutische Zusammensetzung nach einem der Ansprüche 1 bis 15 zur Verwendung bei der Behandlung von chronischer Nierenerkrankung.

## Revendications

1. Une composition pharmaceutique comprenant:
a) un polymère d'amine aliphatique granulé humide choisi dans le groupe formé par le chlorhydrate de Sevelamer et le carbonate de Sevelamer obtenu par emploi d'une solution éthanol/eau comme solution de granulation; et
b) au moins un excipient pharmaceutique;
la solution éthanol/eau étant une solution d'environ 82% à environ 95% d'éthanol (95%) et d'environ 5% à environ 18% d'eau.

2. La composition pharmaceutique selon la revendication 1. dans laquelle la solution éthanol/eau est une solution formée de 95% d'éthanol (95%) et 5% d'eau.

3. La composition selon la revendication 1, dans laquelle les polymères d'amine aliphatique granulés humides sont granulaéspar pulvérisation.

4. La composition pharmaceutique selon la revendication 3, dans laquelle le polymère d'amine aliphatique est le chlorhydrate de Sevelamer .

5. La composition pharmaceutique selon la revendication 3 ou 4, dans laquelle le chlorhydrate de Sevelamer est pratiquement non-hydraté et dont le taux d'hydratation est semblable à celui du taux d'hydratation du chlorhydrate de Sevelamer brut.

6. La composition pharmaceutique selon une quelconque des revendications 3-5, dans laquelle la quantité de chlorhydrate de Sevelamer est comprise entre 60% et environ 95% du poids de la composition.

7. La composition pharmaceutique selon une quelconque des revendications précédentes, dans laquelle la composition est sous forme pharmaceutique.

8. La composition pharmaceutique selon la revendication 7, dans laquelle la forme pharmaceutique est celle d'un comprimé..

9. La composition pharmaceutique selon la revendication 8, dans laquelle le comprimé présente une teneur en eau, déterminée par la méthode d'analyse de Karl Fischer, inférieure à 7%.

10. La composition pharmaceutique selon la revendication 8 ou 9, dans laquelle le comprimé présente une dureté d'au moins environ 17 SCU.

11. La composition pharmaceutique selon la revendication 8, 9 ou 10, dans laquelle le comprimé présente une friabilité d'au moins environ 1,0%.

12. La composition pharmaceutique selon une quelconque des revendications 8-11, dans laquelle le comprimé présente une durée de désintégration dans HC1 0,1N inférieure à environ à 5 minutes.

13. La composition pharmaceutique selon une quelconque des revendications 8-12, dans laquelle le polymère d'amine aliphatique est le chlorhydrate de Sevelamer.

14. La composition pharmaceutique selon la revendication 13, dans laquelle la quantité de chlorhydrate de Sevelamer dans chaque comprimé, calculée sur la base du chlorhydrate de Sevelamer anhydre, est comprise entre 400 mg et environ 800 mg.

15. La composition pharmaceutique selon la revendication 7, dans laquelle la forme pharmaceutique est celle d'un gélule.

16. Un procédé de préparation d'une composition granulaire comprenant des polymères d'amine aliphatique granulés humides comprenant les étapes suivantes de:
a) fourniture d'un polymère d'amine aliphatiques;
b) préparation d'une solution ou le liquide de granulation, où la solution de granulation est une solution ethanol/eau d'environ 82 à environ 95% d'éthanol (95%) et environ 5% à environ 18% d'eau;
c) pulvérisation d'une solution ou le liquide de granulation sur les polymères d'amine aliphatiques et formation d'un granulat par pulvérisation; et
d) mélange du granulat obtenu par pulvérisation avec un ou plusieurs excipients pour former un mélange final.

17. Le procédé selon la revendication 16, dans laquelle la solution de granulation est une solution éthanol/eau préparée à partir de 95% d'éthanol (95%) et 5% d'eau.

18. Le procédé selon une quelconque des revendications 16-17, dans laquelle la solution ou le liquide de granulation comprend en outre un matériau formant liant.

19. Le procédé selon la revendication 18, dans laquelle le matériau formant liant est dissous dans la solution ou le liquide de granulation.

20. Le procédé selon une quelconque des revendications 19, dans laquelle le matériau formant liant comprend de la povidone.

21. Le procédé selon une quelconque des revendications 16-20, dans laquelle le ou excipients comprennent de l'hydroxypropyl cellulose faiblement substituée, une huile végétale hydrogénée et leurs combinaisons.

22. Le procédé selon une quelconque des revendications 16-21, dans laquelle la pulvérisation du liquide de granulation est mise en oeuvre dans un appareil à lit fluidisé.

23. Le procédé selon la revendication 22, dans laquelle la pulvérisation du liquide de granulation est réalisée par un procédé de pulvérisation consistant en pulvérisation par le haut, pulvérisation par le bas et projection de poudre/tangentielle.

24. Le procédé selon la revendication 23, dans laquelle le procédé de pulvérisation est le procédé de pulvérisation par le haut.

25. Le procédé selon la revendication 16, comprenant en outre l'étape e) de compression du mélange final en comprimés.

26. Le procédé selon la revendication 25, comprenant en outre l'étape e) de revêtement des comprimés.

27. Le procédé selon la revendication 16. comprenant en outre l'étape e) de remplissage des gélules par le mélange final.

28. Le procédé une quelconque des revendications 16-27, dans laquelle le polymère d'amine aliphatique consiste en chlorhydrate de Sevelamer ou carbonate de Sevelamer.

29. Le procédé selon la revendication 28, dans laquelle le polymère d'amine aliphatique est le chlorhydrate de Sevelamer .

30. Utilisation du chlorhydrate de Sevelamer pour la fabrication d'une composition pharmaceutique tel que définie dans une quelconque des revendications 1-15 pour le traitement des maladies rénales chroniques.

31. Une utilisation selon la revendication 30, dans laquelle le traitement chlorhydrate de Sevelamer contrôle le sérum phosphate.

32. Une composition pharmaceutique selon une quelconque des revendications 1-15 destinée à être utilisée pour le traitement des maladies rénales chroniques.
